Europäisches Patentamt

European Patent Office

Office européen des brevets

(11) Publication number: **0 198 769**
**A2**

# EUROPEAN PATENT APPLICATION

(21) Application number: 86400767.9

(22) Date of filing: 10.04.86

(51) Int. Cl.4: **A61K 9/22**

(30) Priority: 12.04.85 US 722832

(43) Date of publication of application:
22.10.86 Bulletin 86/43

(84) Designated Contracting States: · . . . .
CH DE FR GB IT LI NL SE

(71) Applicant: FOREST LABORATORIES, INC.
150 East 58th Street
New York, N.Y. 10155-0015(US)

(72) Inventor: Bolton, Sanford
67 Phelps Avenue
Creskill, NJ 07626(US)
Inventor: Desai, Subhash
179-15 Grand Central Parkway
Jamaica, NY 11432(US)

(74) Representative: Bloch, Robert et al
6, rue du Faubourg Saint-Honoré
F-75008 Paris(FR)

(54) Floating sustained release therapeutic compositions.

(57) Sustained release tablets which will float on gastric juice are described. They comprise a hydrocolloid gelling agent such as agar, a therapeutically acceptable inert oil, the selected therapeutic agent and water.

EP 0 198 769 A2

This invention relates to therapeutic compositions in dosage unit form which are capable of floating on gastric juice and delivering their contained therapeutic agent over an extended period of time.

The convenience of administering a single dose of a medication which releases active ingredients over an extended period of time as opposed to the administration of a number of single doses at regular intervals has long been recognized in the pharmaceutical arts. The advantage to the patient and clinician in having consistent and uniform blood levels of medication over an extended period of time are likewise recognized.

The conventional approaches to sustained release can be disadvantageous when the medicament is administered orally because certain classes of active ingredients are not suited to absorption during passage through the gastro-intestinal tract due to their physiochemical properties and/or favorable sites of absorption. Penicillin for example is fully absorbed at one point in the intestine. Once the dosage unit containing penicillin passes this point under the influence of peristaltic movement, the remaining penicillin is not absorbed into the blood stream, but is merely excreted.

Most medicaments will undergo varying degrees of change in solubility by passage from the strongly acid conditions of the stomach to the neutral and to the alkaline conditions of the intestines. Additionally there are medicaments, e.g. antacids which are intended to act in the stomach and therefore lose most beneficial properties when they pass into the intestine.

The advantages of sustained release dosage forms, which are retained in the stomach, for example by floating in the gastric juice, slowly releasing their therapeutic contents into the gastric juice for passage through the intestinal tract will be readily apparent. These include (1) increased contact time for local activity in the stomach where such is required, as in the treatment of stomach ulcers, (2) increased and more efficient absorption for drugs such as penicillin which have specific absorption sites, and (3) the ability to limit the number of dosages.

Many attempts have been made to provide therapeutic dosage forms which will float on the gastric juice and have sustained release capabilities. A series of United States Patents 4 126 672; 4 140 755 and 4 167 558 describes compressed tablets and capsules containing hydrophilic colloids as carriers for the therapeutic agents. The products are said to be in hydrophilic balance so that when they are first ingested they sink to the bottom of the gastric juice. As they absorb water they become more buoyant, rise to the top and float. However, once the tablet is at the lower end of the stomach, its rise to the top might be impeded by food particles in the stomach, or it might pass through the pylorus into the intestine before it attains sufficient buoyancy to float.

Nakano et al in a series of papers, J. Pharm. Pharmacal., 31, 869 (1979); Chem. Pharm. Bull., 28(10), 2905 (1980); and Chem. Pharm. Bull., 27 - (11), 2834 (1979) have described sustained release therapeutic beads formed from rather concentrated agar solutions containing a therapeutic agent. There is no description of the density of the beads or whether they will float.

United States Patent 4 434 153 to Urquahart and Theeuwes describes slow release tiny pills which are slow release because they are coated and embedded in a hydrophilic matrix which swells in contact with water.

Mitra in United States Patent 4 451 260 describes a flexible, thin, water soluble inert carrier sheet or film which contains a drug. The sheet may have an additional barrier on one or both sides. Air spaces are introduced during the manufacturing process causing the material to become buoyant. The sheets are made of synthetic polymers and various excipients may be incorporated to effect dissolution or release of the drug. The matrix does not swell but is flexible. The drug is administered by cutting of an appropriate piece of the film and folding in into a capsule, when the capsule dissolves, the sheet is left to float on the gastric fluids.

Despite the major efforts that have been applied to the problem no completely satisfactory products have yet been produced.

Therapeutic dosage unit forms have now been discovered which are easy to prepare, provide sustained release of contained therapeutic agents and float on gastric juice. These tablets, although not compressed, have sufficient mechanical stability so that they will stand up to the normal stress of production, packaging and dispensing. They have a density which is less than one and sufficiently low so that they will float on gastric juice. Typically the density is from about 0.6 to 0.95.

The tablets contain as essential ingredients, the therapeutic agent in sufficient concentration to be therapeutically effective, a gelling agent, a therapeutically acceptable inert oil and water.

The optimum concentration of the therapeutic agent in the final product will of course vary with the identity of the agent and its optimum therapeutic dosage. Generally the amount of the agent in the dry tablet will be from about 50 to 75 % by weight based on the total weight. (All percentages by weight in this disclosure and claims are based on total weight). It will be apparent to those skilled in the art that there can be appreciable variation from these parameters expecially the range of therapeutic agent without undue effect.

Theophylline is a typical drug which can be usefully dispensed from the tablets of this invention. It is a bronchodilator which has been used for the management of chronic asthma for prophylactic purposes. Its plasma half like has been reported to be 4.4 to 6.2 hours. In order to obtain and retain effective plasma theophylline concentrations, a dose of 200 to 300 mg of theophylline is administered three or four times daily. The rapid absorption and elimination characteristics of theophylline results in large variations in plasma theoyphylline concentration during treatment of patients receiving chronic therapy. Therefore it is a prime candidate for the sustained release, floating tablets of this invention.

Except for those which must be protected from the gastric juice, there is practically no limitation to the therapeutic agents which can be administered in accordance with this invention. They include, for example, analgesics, anorexics, antacids, antibiotics, antidiabetics, antihistamines, steroids, antinauseants, antispasmodics, cardiovascular preparations, decongestants, diuretics, geriatrics, muscle relaxants, tranquilizers and vitamins. More specific examples include acetaminophen, ampicillin, atropine, penicillin, tetracycline, chlorathiazide, phenytoin, riboflavin, quinidine, cemetidine, indomethicin, prednisolone and estradiol. The agents can be employed as free bases or as metal or acid addition salts.

The preferred gelling agent is agar although other gelling agents may be used. These include for example, agarose, carageenin, konjac gum, alginic acid and its salts, cellulose derivatives, carbopol and starch. The concentration of gelling agent in the final .product is about 0.5 to 2 % by weight. Mixtures of gelling agents may be employed.

The preferred therapeutically acceptable inert oil is mineral oil, specifically light mineral oil which ordinarily has a density of from 0.828 to 0.880. Other hydrocarbon or vegetable oils can also be employed. Inert waxes may also be useful. The concentration of the oil in the initial mixture before gelling is from about 8 % to 30 %. The term "inert" means chemically inert, that is it does not react with any of the components of the tablet.

The finished products may also contain other conventional additives such as thickening agents, surfactants, preservatives, bulking agents or antioxidants.

The tablets of this invention are sustained release dosage units, that is they release their medicaments over an extended period of time. The actual rate of release varies with the amount of exposed surface area, and therefore with size and shape of the tablet. The tablets have a density less than one and will float on gastric juice in vivo. Typically, the density is from about 0.6 to 0.95.

Typically, the concentration of the components in the final product as it is provided in dosage unit form is : gelling agent, 0.5 to 4%; oil 12 to 20 %; therapeutic agent 50 to 75 %; balance water.

The tablets of the invention are typically prepared by the following steps :

1. Prepare a solution of the hydrocolloid gelling agent in warm water.

2. Add the selected therapeutic agent to the selected oil.

3. Mix 1 and 2 and cool but not to the point where gelation takes place. This can be determined separately by simple testing.

4. Pour the emulsions which result from the cooling and stirring into tablet molds and let stand until the gel forms.

5. Dry.

Several variations of this procedure are possible as will be readily apparent to those skilled in the art.

During the gelation and drying steps most of the water evaporates. The resulting product, although it is not compressed, is a hard tablet in the shape of the mold. Its compression strength is comparable to that of most commercially available therapeutic tablets. It is characterized by a network of multitudinous air holes and passages.

The concentration of the various compnents in the starting mixture is 0.5 to 2 % gelling agent, 8 to 15 % therapeutically acceptable inert oil, 25 to 50 % therapeutic agent, balance water. As indicated above, the exact amount of therapeutic agent may vary appreciably.

With agar, the solution temperature is about 70°C to 100°C and the pour temperature is about 50°C to 70°C. These are generally the same temperature ranges employed with other gelling agents.

It is surprising to find that such small quantities of gelling agents are capable of forming such rugged tablets without compression.

The following examples are given by way of illustration only and are not to be considered limitations of this invention, many apparent variations of which are possible without departing from the spirit or scope thereof.

EXAMPLE

Nine grams of theophylline was mixed with 2 grams of light mineral oil in a beaker and stirred. In a separate beaker 10 ml of water was added to 0.2 gram of agar, stirred with a magnetic stirrer and heated to boiling. The mixture was cooled 70°C and gradually added to the theophylline-oil mixture with vigorous stirring to form an oil water emulsion.

| Theophylline | 9 grams |
|---|---|
| Light Mineral Oil | 2 grams |
| Water | 10 ml |
| Agar | 0.2 gram |

EXAMPLE 2

Example 1 was repeated to produce the following Formulation 2.

| Theophylline | 9 grams |
|---|---|
| Light Mineral Oil | 3 grams |
| Water | 10 ml |
| Agar | 0.2 gram |

EXAMPLE 3

The density of ten tablets prepared from Formulation 2 was carefully measured. It varied from 0.6943 to 0.7483. The average density was 0.7178. The average weight of the dried tablets was 317 mg, and each contained about :

| Theophylline | 237 mg |
|---|---|
| Light Mineral Oil | 60 mg |
| Agar | 5.3 mg |
| Water | 14.7 mg |

The warm emulsion was poured into a mold plate with plurality of hollow cylindrical molds each with a height of about 0.46 cm and a diameter of about 1.10 cm. The compositions were allowed to cool and gel (about 5 minutes). The tablets were removed from the mold and dried. The density of the tablets varied slightly amongst themselves but the average was about 0.70. This product, Formulation 1, had the following starting composition.

EXAMPLE 4

The dissolution of theophylline from tablets formed from Formulations 1 and 2 was determined by the standard USP Basket method at 50 RPM. The results are shown below where the values given are percent release of theophylline.

| Time | 1 | | 2 | |
| --- | --- | --- | --- | --- |
| | pH 1.2 | 7.4 | pH 1.2 | 7.4 |
| 1 | 23.8 | 26.1 | 21.0 | 20.4 |
| 2 | 31.4 | 36.7 | | |
| 3 | | | 38.5 | 37.7 |
| 4 | 43.4 | 50.3 | | |
| 6 | | | 54.8 | 53.4 |
| 8 | 65.1 | 65.9 | | |
| 12 | 74.5 | | 75.9 | 74.9 |
| 13 | | 80.0 | | |
| 19 | | | 91.3 | 91.2 |

It is apparent from these values that the tablets release their theophylline content over an extended period of time.

EXAMPLE 5

Plasma concentrations of theophylline in a human adult male volunteer were determined after ingestion of a single 237 mg tablet of Formulation 2. The values are given below.

| TIME (hours) | CONCENTRATION (mcg/ml) |
| --- | --- |
| 1 | 0 |
| 3 | 1.7 |
| 6 | 1.95 |
| 12 | 2.7 |
| 18 | 3.75 |
| 24 | 3.2 |

The sustained release effect is evident from the figures.

**Claims**

1. A therapeutic composition in dosage unit from as a non-compressed tablet having a density of less than one and capable of floating on gastric juice _in vivo_ characterized in that it comprises a matrix formed from a gelling agent containing a therapeutic agent, a therapeutically acceptable inert oil and water in the following percentages by weight based on the total weight : gelling agent, 0.5 to 4 %; oil, 8 to 30 %; therapeutic agent 50 to 75 %; balance water.

2. A therapeutic composition according to claim 1 comprising an agar matrix containing theophylline, mineral oil and water in the following percentages by weight based on the total weight; agar, 0.5 to 4 %; oil, 12 to 20 %; theophylline 50 to 75 %; balance water.

3. A method of forming a therepeutic composition according to claim 1 characterized in that it comprises :

(1) forming a solution of a gelling agent in water,

(2) forming a first mixture containing a therapeutic acceptable inert oil and a therapeutic agent,

(3) cooling said solution,

(4) combining the solution and mixtures to produce a second mixture containing from 0.5 to 2 % of the gelling agent, 8 to 30 % of the oil, 25 to 50% of the therapeutic agent, balance water, all by weight based on the total weight,

(5) pouring the second mixture into a tablet mold and letting it stand in the mold to form a gel, and

(6) drying.

4. A method of forming a therapeutic composition according to claim 2 characterized in that it comprises :

(1) forming a solution containing agar in water,

(2) forming a first mixture containing theophylline in mineral oil,

(3) cooling said solution,

(4) combining the solution and mixture to produce a second mixture containing from 0.5 to 2 % agar, 8 to 15 % of the oil and 25 to 50 % of the theophylline, balance water, all by weight based on the total weight,

(5) pouring the second mixture into a tablet mold and letting it stand in the mold to form a gel, and

(6) drying.